# EUROPEAN PATENT APPLICATION

(11) **EP 2 705 893 A1**
(43) Date of publication of application: **12.03.2014**
(21) Application number: 12183037.6
(22) Date of filing: 05.09.2012
(51) Int. Cl.: B01D 53/14, C01B 25/10, C07C 17/38, C01B 7/07, C01B 7/09, C01B 7/19

(54) **Process for separating acid-containing gas mixtures**

(71) Applicant: Solvay SA, 1120 Bruxelles (BE)
(72) Inventor: Fischer, Reiner, 38173 Dettum (DE)
(74) Representative: Mross, Stefan P.M.

(57) **Abstract**

A process for reversibly removing at least one acid from a gas mixture comprising at least one acid and at least one of a carbonyl dihalide, a phosphoryl trihalide, a phosphorous pentafluoride, and an acid halide is provided. The process comprises contacting the gas mixture with a solvent comprising a sulphone compound.

## Description

### Technical field

The present disclosure relates to a process for reversibly removing at least one acid from a gas mixture comprising at least one acid and at least one of a carbonyl dihalide, a phosphoryl trihalide, phosphorous pentafluoride, and an acid halide, as well as to the use of a solvent for reversibly removing at least one acid from such a gas mixture.

### Background art

Acids such as HCl, HF and HBr, either alone or in combination, are involved in many chemical reactions, be it as starting materials, as reaction products or as products of a side reaction, and may be removed from gas mixtures containing these acids by separating agents such as amines. However, amine-hydrohalide adducts are obtained which have to be dumped or burned. Sometimes, other constituents of the gas mixture may be sensitive towards or reactive with bases. In some gas mixtures, the boiling points of the gas constituents may be so close that distillation is unsuitable to remove HCl or other acids.

Carbonyl fluoride is a gas which can be used as etching gas for manufacturing semiconductors and for cleaning CVD chambers. International patent application WO 05/085129 A2 describes a photochemical process for the manufacture of C(O)F₂ from CHClF₂. The process described therein provides for the preparation of C(O)F₂ by photo-oxidation of CHClF₂ with oxygen. Light having a spectral range covering at least 50 nm, in the sense that the light having the shortest wavelength and the light having the longest wavelength are at least 50 nm apart, is radiated in.

Phosphoryl fluoride is a gas which can be prepared by fluorination of phosphoryl chloride with metallic fluorides. As described in international patent application WO2012/016924 A1, phosphoryl fluoride can be reacted with Li₃PO₄ to form LiPO₂F₂ which is useful as electrolyte salt or electrolyte salt additive in Li ion batteries and other devices suitable for storing electric energy.

Phosphorous pentafluoride is an intermediate for preparing electrolyte salts for lithium ion batteries. For example, phosphorous pentafluoride can be reacted with lithium fluoride to form lithium hexafluorophosphate. Phosphorous pentafluoride can be prepared from phosphorous pentachloride or phosphorous trichloride and chlorine and hydrogen fluoride. HCl is formed in this reaction and must be separated off from phosphorous pentafluoride.

Organic gaseous acid fluorides such as CF₃C(O)F or CHF₂C(O)F are starting compounds for fluorinated organic compounds, for example for fluorovinyl ethers, which in turn are comonomers used in the production of resins or elastomers. CH₃C(O)F is useful as a pesticide and as an etching agent for silicon related compounds, while CHF₂C(O)F is an intermediate in the manufacture of finishes for textiles. Acid fluorides may contain HCl or HBr depending on the method used for preparing the acid fluorides. Sometimes, acid fluorides may further comprise HF.

Gas mixtures comprising HF may be, for example, result of a fluorination reaction with hydrogen fluoride or fluorine. For example, carboxylic acid fluorides can be prepared by the reaction of carboxylic acid chlorides and HF or by photochemical oxidation, as described in US patent US 6 489 510 B1. Such gas mixtures may further comprise HCl.

There is the need of separating gas mixtures comprising HCl and a carboxylic acid fluoride, or HF and a carboxylic acid fluoride, or HF, HCl and a carboxylic acid fluoride, or HCl and PF₅, or HCl and C(O)F₂, or HCl and POF₃, respectively. It is also generally desirable to separate HF, HCl and HBr from gas mixtures comprising HF, HCl and/or HBr and other gas constituents such as carbonyl dihalides, phosphoryl trihalides, phosphorous pentafluoride, and acid halides, or to separate these gas mixtures so as to obtain gas mixtures or pure gases which are depleted of HF, HCl and HBr, respectively, and in which the other constituent(s) is(are) thus concentrated. Further, it can be desirable to remove the acid from the gas mixtures mentioned above under water-free conditions, for example, to prevent corrosion damages in an apparatus or to reduce the amount of contaminated washer water used for acid removal.

International patent application WO 2006/045518 A1 describes a process for the removal of HCl, HF or HBr from mixtures with C(O)F₂, PF₅ or carboxylic acid fluorides by means of ionic liquids. Several ionic liquids are disclosed as suitable separating agents.

US patent application US 2007/0293707 A1 describes a process for separating HCl and COCl₂ comprising bringing a mixture of HCl and COCl₂ into contact with an ionic liquid.

International patent application WO 2007/125065 A1 describes gas mixtures comprising acids such as HF, HCl or HBr and other constituents, in particular gas mixtures comprising or consisting of carboxylic acid fluorides, C(O)F₂ or phosphorous pentafluoride and HCl and optionally HF, which are separated by ionic liquids. The process is performed reversibly.

However, on the one side, the choice of ionic liquids is limited and, on the other side, ionic liquids may be sensitive to or reactive with some acids, such as for example HCl, HF or HBr, and may not ensure the attainment of the desired degree of purification of the gas mixture from the acids. Furthermore, ionic liquids are expensive.

### Summary of the disclosure

An object of the present disclosure is to provide a process for reversibly removing at least one acid from a gas mixture comprising at least one acid and at least one of a carbonyl dihalide, a phosphoryl trihalide, a phosphorous pentafluoride, and an acid halide, which is effective, results in an enhanced degree of purification and has a wide operability range, in particular as to the choice of the separating agent.

According to one aspect thereof, the present disclosure provides a process for reversibly removing at least one acid from a gas mixture comprising at least one acid and at least one of a carbonyl dihalide, a phosphoryl trihalide, a phosphorous pentafluoride, and an acid halide, the process comprising contacting the gas mixture with a solvent comprising a sulphone compound. The solvent acts as a separating agent.

According to one embodiment, the at least one acid comprises at least one of HCl, HF and HBr. The at least one acid may consist of at least one of HCl, HF and HBr.

The content of HCl, HF or HBr may vary depending on the source of the gas mixture and any pre-treatment steps, such as for example adsorption. Generally, the amount of HCl, HF or HBr in the gas mixture may be in the range from 100 ppm by weight or even less to 99 % by weight.

If the gas mixture which has to be treated comprises HCl and, for example, COF₂, CF₃C(O)F or CHF₂C(O)F₂, and originates from a photo oxidation reaction, the content of HCl may be relatively high : in such cases, the content of HCl may be for example in the range from 20 % to 70 % by weight.

The gas mixture to be treated may also originate from a chlorine-fluorine exchange reaction wherein HF is used as reactant. For example, the gas mixture may comprise COF₂ and HCl from the reaction between COCl₂ and HF, or it may contain PF₅ from the reaction of PCl₃, Cl₂ and HF. Also in such mixtures, the content of HCl may be relatively high : in such cases, the content of HCl may be for example in the range of 20 % to 70 % by weight.

According to one embodiment, the at least one acid comprises HCl and HF. The at least one acid may consist of HCl and HF. Gas mixtures originating, for example, from chlorine-fluorine exchange reactions, such as those mentioned above, *e.g*. the manufacture of COF₂ or PF₅ using HF as reactant, may comprise significant amounts of HCl and traces up to significant amounts of HF. The content of HCl often will be in the range of 20 % to 70 % by weight.

The content of HF may depend on the respective reaction conditions, such as for example on the ratio between HF and the chlorine atoms to be substituted and the conversion of the starting material. A chlorine-fluorine exchange reaction may be performed using HF, in view of the chlorine atoms to be substituted, in substoichiometric ratio, in a stoichiometric ratio, or even in excess. In the first two alternatives, the HF content may be for example in the range of 100 ppm (or even less) to 10 % by weight. If HF is used in excess, the content of HF in the gas mixture may be higher. For example, the gas mixture may originate from a chlorine-fluorine exchange reaction, and may comprise COF₂, PF₅, CF₃C(O)F or CHF₂C(O)F, HCl and 1 % to 70 % by weight of HF.

According to one embodiment, the at least one acid may consist of HCl and of traces of HF. Such acids may be formed starting from reaction mixtures initially comprising, for example, only HCl and COF₂, PF₅, CF₃C(O)F or CHF₂C(O)F. Since said fluorides are susceptible to hydrolysis, a trace amount of HF may be formed. The term "trace amount" is used to indicate an HF content of, for example, from 10 ppm to 5,000 ppm in the reaction mixture.

The carbonyl dihalide may comprise one or more carbonyl dihalides. In an analogous manner, the phosphoryl trihalide may comprise one or more phosphoryl trihalides, and the acid halide may comprise one or more acid halides.

The carbonyl dihalide may comprise or consist of C(O)F₂.

The phosphoryl trihalide may comprise or consist of POF₃.

The acid halide may comprise or consist of one or more carboxylic acid halides.

For example, the carboxylic acid halide may be carboxylic acid fluoride.

The carboxylic acid fluoride may be selected from CH₃C(O)F, CHF₂C(O)F, CF₃C(O)F, C₂F₅C(O)F and mixtures thereof.

According to one embodiment, the gas mixture may comprise at least one acid and at least one of C(O)F₂, PF₅, POF₃, CH₃C(O)F, CHF₂C(O)F, CF₃C(O)F, C₂F₅C(O)F and mixtures thereof. The gas mixture may consist of least one acid and at least one of C(O)F₂, PF₅, POF₃, CH₃C(O)F, CHF₂C(O)F, CF₃C(O)F, C₂F₅C(O)F and mixtures thereof.

According to one embodiment, the gas mixtures comprise constituents having a boiling point below 50°C at 0.1 MPa abs., which are gaseous at normal conditions (25°C, 0.1 MPa abs.), such as, for example, trifluoroacetyl fluoride, difluoroacetyl fluoride, PF₅, C(O)F₂ and, as acids, HCl, HF and/or HBr.

According to one embodiment, the at least one acid and each of the gas constituents to be separated off have boiling points which do not differ from each other by more than20°C. The at least one acid may have for example the closest boiling point to the boiling point of the gas constituent of the gas mixture to be depleted of the at least one acid.

When the at least one acid comprises HCl, the gas mixture may comprise, for example, constituents having a boiling point of below 50°C at ambient pressure (0.1 MPa abs.), or constituents which are gaseous at normal conditions (25°C, 0.1 MPa abs.), such as carboxylic acid chlorides like trifluoroacetyl chloride (TFAC) or difluoroacetyl chloride (DFAC). HCl may also be removed from carboxylic acid fluorides which are gaseous at normal conditions, for example from difluoroacetyl fluoride, trifluoroacetyl fluoride, C₂F₅C(O)F or CH₃C(O)F. Also HBr may be removed, when the at least one acid comprises HBr. According to one embodiment, carbonyl fluoride or carboxylic acid chlorides and carboxylic acid fluorides with at most 3 carbon atoms may be purified. Also HF may be removed, when the at least one acid comprises HF.

According to one embodiment, the at least one acid comprises HCl and optionally HF and may be removed, for example, from any of C(O)F₂, CHF₂C(O)F, CF₃C(O)F and PF₅, either alone or in combination.

According to one embodiment, carbonyl fluoride is treated. Carbonyl fluoride may be for example treated to be purified from gas mixtures containing carbonyl fluoride and HCl, HF or both.

As separating agent of the process, a solvent comprising a sulphone compound is used. This solvent is not sensitive to or reactive with acids, such as for example to HCl, HF or HBr, and results in an increased degree of purification of the gas mixture from the acids.

In the present description and in the following claims, a sulphone compound is a compound comprising a sulphone group.

According to one embodiment, the sulphone compound comprises a tetrahydrothiophene 1,1-dioxide compound.

The tetrahydrothiophene 1,1-dioxide compound may have for example the following formula : 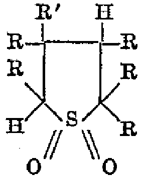 wherein each R is selected from hydrogen, methyl and ethyl radicals, and R' is selected from alkoxy radicals having 1-5 carbon atoms.

In one embodiment, the tetrahydrothiophene 1,1-dioxide compound is tetrahydrothiophene 1,1-dioxide, i. e. sulfolane. However, other tetrahydrothiophene 1,1-dioxide compounds may be used as solvents, such as for example tetrahydro-2-methylthiophene 1,1-dioxide, tetrahydro-3-methylthiophene 1,1-dioxide, tetrahydro-2-ethylthiophene 1,1-dioxide, tetrahydro-3-ethylthiophene 1,1-dioxide, tetrahydro-2,5-dimethylthiophene 1,1-dioxide, tetrahydro-3,4-diethylthiophene 1,1-dioxide, tetrahydro-3-methyl-2,5-diethylthiophene 1,1-dioxide, tetrahydro-2,3,4,5-tetramethylthiophene 1,1-dioxide, tetrahydro-2,2,3,4,5-pentaethylthiophene 1,1-dioxide, tetrahydro-3-methoxythiophene 1,1-dioxide, tetrahydro-3-ethoxythiophene 1,1-dioxide, tetrahydro-2-methyl-3-isopropoxythiophene 1,1-dioxide, tetrahydro-2-ethyl-3-methyl-4-tert-butoxythiophene 1,1-dioxide, tetrahydro-2,2,3,4,5-pentaethyl-4-pentoxythiophene 1,1-dioxide and the like, and mixtures thereof.

According to the present disclosure, mixtures of solvents may be used, for example mixtures of two, three or more different solvents, for example selected among one or more of the above-mentioned tetrahydrothiophene 1,1-dioxide compounds. In this way, the separation properties, for example the polarity or the affinity to the gas constituent(s) to be separated, or the viscosity or the temperature at which absorption is achieved, can be adjusted.

According to one embodiment, contacting the gas mixture with the solvent is performed at a predetermined temperature, for example in the range of -40°C to 150°C. According to one embodiment, the temperature may range from 0°C to 50°C, and for example from 15°C to 30°C.

According to one embodiment, contacting the gas mixture with the solvent is performed at a predetermined pressure, for example at a pressure at which, under the respective temperature, all components of the gas mixture are in a gaseous or vapor state. The contact may be for example performed in the range from 0.05 MPa abs. to 5 MPa abs. However, the pressure may be even higher, in which casea pressure-resistant apparatus may be used. The pressure may for example range from ambient pressure (0.1 MPa abs.) to 1 MPa abs. or higher, and for example may be ambient pressure.

The process may further comprise recovering the at least one acid. Recovering the at least one acid may be performed by heating, optionally under vacuum. Heating may be performed at a temperature of at least 50°C, and for example at a temperature from 50°C to 280°C.

According to one embodiment, the gas mixture is obtained by photochemical oxidation of CHClF₂ for the preparation of C(O)F₂.

In this embodiment, the process may further comprise separating any unreacted CHClF₂, for example by distillation.

Independently from the way in which the gas mixture is obtained, according to the present disclosure, the at least one acid is reversibly absorbed in the solvent. Thus, according to one embodiment, the process may further include desorption of the reversibly absorbed acid(s). In such an embodiment, the process comprises an absorption step and a desorption step. Consequently, after desorption of the reversibly absorbed acid, the solvent may be used again for acid removal or for any other purpose.

According to one embodiment, the sequence of absorption step and desorption step is performed twice. However, the sequence of absorption step and desorption step may be performed more than twice, for example, three times, four times or five times or a greater number of times, for example fifty times, hundred times or more. The number of sequences of absorption step and desorption step depends on the content of impurities in the raw gas mixture and on the degree of desired depletion.

The process may be performed in a continuous manner in two or more apparatus, a first one or a first group of which may be used for absorption, a second one or a second group of which may be used for desorption.

According to one embodiment, the gas mixture may be subjected to a plurality of absorption steps, thus passing more than once through the solvent.

According to one embodiment, the process further comprises recycling the solvent and contacting the gas mixture which has been already contacted with the solvent with the recycled solvent to further deplete the content of acid.

According to this embodiment of the process, the sequences of absorption step and desorption step may be performed with the same gas mixture. In this way, the gas mixture becomes more and more depleted of the at least one acid.

An intermediate desorption step of the acid may be provided between two absorption steps.

The gas mixture may be treated in a plurality of absorption steps, and the solvent may be recycled a plurality of times.

According to one embodiment, a raw, untreated gas mixture may be treated with a recycled solvent. According to this embodiment of the process, fresh gas mixture may be treated in an absorption step, the solvent may be desorbed from acid, and then, again, fresh gas mixture may be treated, and afterwards, again a desorption step may be performed. In this embodiment, the gas mixture may be treated only in one absorption step, but the solvent is applied two or more times after the desorption step.

According to one embodiment, the raw gas mixture may be treated two or three or more times with the solvent. By way of exemplary illustration, with reference to a case of a carbonyl fluoride/HCl raw gas mixture comprising more than 40 % by weight of HCl, carbonyl fluoride with an HCl content of less than 1000 ppm and even less, depending on the number of absorption steps, can be obtained.

The absorption step(s) may be performed in a common absorption apparatus suitable for gas-liquid contact, for example in a reactor, for example in a counterflow reactor.

If desired, the process may comprise two or more consecutive absorption steps. The solvent used in the consecutive absorption steps may be collected from the absorption apparatus and treated in a desorption step in a desorption apparatus to remove the entrained acid. The desorption step may comprise applying vacuum and/or heating. The desorbed solvent may be then recycled to the absorption apparatus, and additional gas mixture may then be treated.

According to one embodiment, the process is performed in the absence of water. According to this embodiment, the solvent is essentially water-free. The solvent is essentially water-free if the solvent comprises less than 0.1 % by weight of water. In this way, any possible hydrolytic reaction takes place only to a minor extent, if at all. In any case, if such a minor amount of water is present, the content thereof may be further reduced by hydrolytic reactions if, for example, gas mixtures comprising COF₂, POF₃, PF₅ or acid fluorides are treated. If desired, initial fractions of product leaving the solvent which is undesirably contaminated by hydrolytic reaction products may be dumped.

The process may be applied to any gas mixture as defined in claim 1 from which the at least one acid may be removed in a water-free process.

The contact between the gas mixture and the solvent may be performed according to conditions which are common in gas-liquid operations. For example, the gas mixture to be treated may be passed through the solvent. The contact surface may be enhanced by injectors, frits or mixing equipment provided in a separation apparatus. For example, the process may be performed in a bubble column, in a column with bubble cap trays or in a packed column.

The at least one acid, for example HCl, HF and/or HBr, is retained in the solvent, and constituents such as C(O)F₂, PF₅ or any other gas constituents which are not absorbed pass through the solvent. When a desired purity is attained, the gases may be liquefied and used for respective purposes.

In some gas mixtures, further constituents may be comprised which also pass through the solvent. For example, gas mixtures comprising C(O)F₂ and HCl deriving from the photochemical oxidation of CHClF₂ may contain unreacted starting material, *i.e.* CHClF₂. Constituents of the gas mixtures which are not retained in the solvent may be removed before passing the gas mixture through the solvent, for example by a preliminary fractionated distillation or condensation. Alternatively, such constituents may be left in the gas mixture and then may be removed after the contact with the solvent, for example by fractionated distillation or condensation. A pressure distillation to remove low and high boiling substances may be performed after one or each absorption step, if a plurality of absorption steps is envisaged. A pressure distillation may be performed before or after performing the process of the disclosure.

The constituents retained in the solvent may be desorbed from the solvent physically, in at least one desorption step. Desorption may be performed by a change, for example an increase, of the temperature, by a change, for example a decrease, of the pressure and/or by passing an inert gas, such as for example nitrogen or argon, through the solvent. A pressure lower than the pressure in the absorption step may be set : for example, vacuum may be applied. Additionally, or instead of lowering the pressure, the temperature may be increased above the temperature of the absorption step, for example, to a range from 50°C to 300°C. Optionally, the temperature and the pressure may be both changed. By desorption and removal of the constituents of the gas mixture from the solvent, the solvent is completely regenerated and may be used again in a new absorption step. According to one embodiment, the temperature during the desorption step is not higher than 100°C, but the temperature may be as high as below the decomposition temperature of the solvent. According to one embodiment, the pressure during the desorption step is not lower than 0.01 MPa (abs.). However, desorption may be performed also at a lower pressure. Desorption may be performed in a predetermined time, for example within 1 to 2 hours, or even faster, depending on the desorption conditions, such as temperature, degree of vacuum or stirring, where present, even at temperatures of or below 100°C down to, for example, 40°C. According to one embodiment, when the desorption step is performed by means of a change of pressure, the pressure is lower than the pressure set during the absorption step. If the contact between the gas mixture and the solvent is performed at overpressure, for example, at 0.5 MPa (abs.) or more, the subsequent desorption step may be performed at a pressure which is at or slightly above the ambient pressure, for example, in the range of 0.1 MPa (abs.) to 0.15 MPa (abs.). However, vacuum may be applied and, optionally, the temperature may be increased to complete reconditioning of the solvent. According to one embodiment, the absorbed constituents are not completely desorbed. For example, only substantial parts of constituents, for example, 80 % by weight or more, may be desorbed. The desorption may be performed batchwise or continuously. A suitable apparatus may be for example a stripper column.

After the desorption step, the at least one acid may be again separated with the solvent so regenerated.

The at least one acid, such as HCl, HF and/or HBr, can be separated in a simple manner from the other constituents of the gas mixture, for example from gaseous carboxylic acid chlorides and carboxylic acid fluorides, such as for example from phosphorous pentafluoride or C(O)F₂, and the solvent may be used many times.

An embodiment of the process provides the possibility of separating HF or HCl from mixtures thereof with C(O)F₂.

However, also different mixtures may be treated, for example mixtures obtained from fluorination reactions of phosphorous-chlorine compounds with HF, for example in the preparation ofPF5 from phosphorous (III) chlorides or phosphorous (V) chlorides and HF and Cl₂ or F₂, respectively.

When the gas mixture comprises HCl as acid and, as further constituents, at least one of CH₃C(O)F, CHF₂C(O)F, CF₃C(O)F, C₂F₅C(O)F, C(O)F₂ and PF₅, the process may result in a purified gas mixture comprising HCl in an amount lower than 50 ppm by weight, for example lower than 15 ppm by weight or less, for example lower than 10 ppm or less.

When the gas mixture comprises HCl as acid and C(O)F₂ and PF₅ as further constituents, the process may result in a purified gas mixture comprising HCl in an amount of equal to or higher than 0.5 ppm by weight, for example in an amount of equal to or more than 1 ppm to 15 ppm. PF₅ may be comprised in the purified PF₅ in an amount of at least 98 % by weight, for example in an amount of at least 99 % by weight. The purified C(O)F₂ may comprise up to 5 % by weight of an inert gas, for example nitrogen, carbon dioxide, argon or helium. The content of C(O)F₂ in the purified gas mixture may be of at least 94.5 % by weight, for example from 94.5 % to 99.9 % by weight, while the inert gas may be in an amount of 0 % to 5 % by weight, and the total acid, including HCl, may be in an amount from 1 ppm to 50 ppm, for example from 1 to 15 ppm. The content of organic impurities, including COFCl, may be less than 1000 ppm, for example less than 500 ppm. The content of other impurities, for example air, may be less than 1000 ppm.

The purified gas mixture may comprise from 99.4 % by weight or more of C(O)F₂, for example from 1 and 15 ppm of acid, including HCl, and for example equal to or less than 0.5 % by weight of inert gas, including CO₂, less than 50 ppm of organic impurities, including COFCl, and less than 150 ppm of other impurities, *e.g*. air.

The purified constituents mentioned above, such as purified carbonyl fluoride, may be obtainable by repeated treatment with a solvent as described above. The number of absorption steps may depend on the degree of contamination of the raw gas mixture.

According to another aspect thereof, the present disclosure provides the use of a solvent comprising a sulphone compound for reversibly removing at least one acid from a gas mixture comprising at least one acid and at least one of a carbonyl dihalide, phosphoryl trihalide, phosphorous pentafluoride, and an acid halide.

Embodiments of such use are based on solvents, acids and gas constituents as defined above with reference to the embodiments of the process.

### Detailed description of embodiments

In the following, embodiments of the process for reversibly removing at least one acid from a gas mixture comprising at least one acid and at least one of a carbonyl dihalide, phosphoryl trihalide, phosphorous pentafluoride, and an acid halide are described.

### Example 1 (comparative)

A reversible removal of HCl from C(O)F₂ by means of EMIM-trifluoromethansulfonate was performed in accordance with Example 2 of WO 2007/125065 A1. A mixture consisting of C(O)F₂ and HCl (volume ratio 2:1) was passed through 1200 g of ionic liquid consisting of 1-ethyl-3-methyl-imidazoliumtrifluormethane sulfonate (EMIM-triflat). In total, 6000 g of the mixture was passed through the separating agent in 10 absorption/desorption steps whereby HCl was selectively absorbed. After each absorption/desorption cycle, a sample of the ionic liquid was analyzed for chloride by ion chromatography. The chloride content was below the detection limit, i. e. below 20 ppb.

### Example 2 (according to the present disclosure)

A reversible removal of HCl and HF from C(O)F₂ by means of sulfolane was performed on a gas mixture comprising C(O)F₂ and HCl in a volume/volume ratio of 2:1 and HF as a minor impurity, i. e. in an amount of 1000 ppm. The gas mixture was passed at approximately 20°C and at ambient pressure through sulfolane. In order to obtain a good dispersion of the gas mixture, the gas mixture was introduced into the solvent via a frit made from Monel metal. HCl and HF were retained in the solvent. COF₂ containing only 2 vol- % of HCl and 100 ppm of HF was withdrawn from the solvent. The content of HCl and HF was further reduced by repeating the treatment at least one further time.

### Example 3 (according to the present disclosure) : recovery of the solvent

Sulfolane comprising HCl and HF was treated in a stripper column at 150°C in a counter current of N₂ to obtain sulfolane essentially free of said acids. Sulfolane obtained in this manner was reused as solvent to separate HCl and/or HF from COF₂.

## Claims

1. A process for reversibly removing at least one acid from a gas mixture comprising at least one acid and at least one of a carbonyl dihalide, a phosphoryl trihalide, a phosphorous pentafluoride, and an acid halide, the process comprising contacting the gas mixture with a solvent comprising a sulphone compound.

2. The process according to claim 1, wherein the at least one acid comprises at least one of HCl, HF and HBr.

3. The process according to claim 1 or claim 2, wherein the carbonyl dihalide is C(O)F₂.

4. The process according to claim 3, wherein the gas mixture is obtained by photochemical oxidation of CHClF₂ for the preparation of C(O)F₂.

5. The process according to claim 4, further comprising separating any unreacted CHClF₂ by distillation.

6. The process according to any of claims 1-5, wherein the phosphoryl trihalide is POF₃.

7. The process according to any of claims 1-6, wherein the acid halide is a carboxylic acid halide.

8. The process according to claim 7, wherein the carboxylic acid halide is a carboxylic acid fluoride.

9. The process according to claim 8, wherein the carboxylic acid fluoride is selected from CH₃C(O)F, CHF₂C(O)F, CF₃C(O)F, C₂F₅C(O)F and mixtures thereof.

10. The process according to any one of claims 1-9, wherein the sulphone compound comprises a tetrahydrothiophene 1,1-dioxide compound.

11. The process according to claim 10, wherein the tetrahydrothiophene 1,1-dioxide compound has the formula : 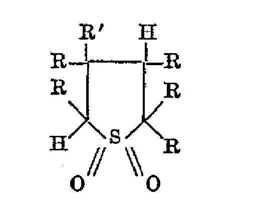 wherein each R is selected from hydrogen, methyl and ethyl radicals, and R' is selected from alkoxy radicals having 1-5 carbon atoms.

12. The process according to claim 11, wherein the tetrahydrothiophene 1,1-dioxide compound is sulfolane.

13. The process according to any one of claims 1-12, further comprising recovering the at least one acid.

14. Use of a solvent comprising a sulphone compound for reversibly removing at least one acid from a gas mixture comprising at least one acid and at least one of a carbonyl dihalide, a phosphoryl trihalide, a phosphorous pentafluoride, and an acid halide.
